# NEW EUROPEAN PATENT SPECIFICATION

(11) **EP 0 131 147 B2**
(45) Date of publication and mention of the opposition decision: **04.12.1996**
(45) Mention of the grant of the patent: 14.09.1988
(21) Application number: 84106343.1
(22) Date of filing: 04.06.1984
(51) Int. Cl.: C07D 499/68, C07D 499/16, A61K 31/43

(54) **Crystalline amoxycillin salt**
Kristallines Amoxycillin-Salz
Sel d'amoxycilline cristallin

(30) Priority: 10.06.1983 US 503012; 12.03.1984 US 588561
(43) Date of publication of application: 16.01.1985
(73) Proprietor: Beecham Group p.l.c., Brentford, Middlesex TW8 9EP (GB)
(72) Inventor: Clark, Dennis Edward, Basking Ridge New Jersey 07920 (US); Windus, Charles Edward, Somerville New Jersey 08876 (US)
(74) Representative: Tyrrell, Arthur William Russell, Dr.

(56) References cited:
- GB-A- 1 241 844
- GB-A- 1 382 409
- GB-A- 1 464 026
- GB-A- 1 465 694
- GB-A- 1 527 557
- GB-A- 1 543 317
- GB-A- 1 552 011
- GB-A- 1 576 731
- GB-A- 2 096 599
- CHEMICAL ABSTRACTS, vol. 81, no. 2, July 15, 1974, p. 149, abstract 6279s, Columbus, Ohio, US

## Description

This invention relates to β-lactam antibiotics and in particular to a salt of the penicillin antibiotic amoxycillin. The amoxycillin salt of this invention is active against Gram-positive and Gram-negative bacteria and is useful as a therapeutic and prophylactic agent against bacterial infections in animals including man. In particular it is useful for parenteral administration.

Although there are numerous known methods for the preparation of the widely used antibiotic, sodium amoxycillin, all such methods have produced an amorphous material.

A process has now been devised which produces sodium amoxycillin in crystalline form. The material is anhydrous, essentially pure, non-hygroscopic and has high stability.

Accordingly this invention provides crystalline sodium amoxycillin.

The invention also provides crystalline anhydrous sodium amoxycillin.

The crystalline sodium amoxycillin of this invention is solvent free. It contains less than 2.5% of solvent, suitably less than 1% and preferably 0.5% or less solvent. The crystalline anhydrous sodium amoxycillin contains less than 2.5% of water, suitably less than 1% and preferably 0.5% or less water.

The invention also provides a process for the preparation of crystalline sodium amoxycillin, which process comprises removing the solvent molecules from a crystalline solvate of sodium amoxycillin, and wherein the said removal of solvent molecules from the solvate may comprise the conversion of the solvate into a different solvate, from which the solvent molecules may be removed more readily, and subsequently removing the solvent molecules therefrom.

The crystalline material of this invention is characterized by a sharp infra-red spectrum and a multiple-line X-ray powder diffractogram.

For the preparation of the product of this invention, a crystalline solvate of sodium amoxycillin is employed as starting material. The solvate may be prepared by any convenient method. In general the solvate is prepared by bringing together a sodium compound, a non-aqueous solution of amoxycillin and a solvent which can form a solvate with sodium amoxycillin preferably a solvent which is labile, i.e. is readily removable from the solvate. The solvate is then precipitated from the solution, for example by precipitation with a solvent in which the solvate is insoluble. An excess of the solvating solvent itself may be sufficient to cause the precipitation. A solvate which is crystalline is preferably isolated in crystalline form by carrying out the precipitation under conditions which allow crystallization to take place.

In one suitable method a solution of amoxycillin is prepared by suspending amoxycillin trihydrate in a suitable solvent or mixture of solvents and adding a strongly basic sodium compound such as sodium methoxide. A suitable solvent system for this stage is methanol or a mixture containing methanol and a solvating solvent. Sufficient solvating solvent is then added under conditions which allow crystallization of the solvate.

Alternatively amoxycillin trihydrate may be dissolved with the aid of an organic base, such as triethylamine and the resultant solution treated with a sodium compound such as sodium methoxide, sodium hydroxide or preferably the sodium salt of an alkanoic acid, e.g. sodium 2-ethyl hexanoate. These steps may conveniently be combined by using a mixture of an organic base and a sodium compound to bring about dissolution of the amoxycillin trihydrate. A suitable solvent system for this stage is methanol or a mixture of methanol and solvating solvent. Sufficient solvating solvent is then added to cause crystallization of the solvate.

Suitable solvents which form solvates with sodium amoxycillin include C₆₋₈ aromatic hydrocarbons such as benzene and toluene; C₃₋₁₀ alkanols, such as n-propanol, isopropanol, n-butanol and t-butanol; C₂₋₁₂ alkyl and cyclic ethers such as diethyl ether, di-isopropyl ether, bis (2-methoxyethyl) ether, 1,2-dimethoxyethane, dioxan and tetrahydrofuran; chlorinated hydrocarbons such as methylene dichloride; C₁₋₆ alkyl esters of C₁₋₆ alkanoic acids, such as methyl formate, ethyl formate, methyl acetate and ethyl acetate; and amides. It is preferable that the solvent employed in the production of the sodium amoxycillin of this invention is pharmaceutically acceptable.

Preferred solvating solvents are tetrahydrofuran, methyl acetate and ethyl acetate. These solvents form solvates from which the solvent molecules are readily removed.

One known class of solvates of sodium amoxycillin comprises amide solvates. Such solvates are particularly stable. Amide solvates of sodium amoxycillin comprise a solvate of sodium amoxycillin with an amide. Such solvates are described in GB 1465694. A particular solvate, with the amide N-methylpyrrolidinone, is described in EP 039967-A.

The amide solvate may be prepared by any convenient method. Preferably a source of sodium ions is added to a mixture or solution of amoxycillin or a salt thereof and the amide.

For example, amoxycillin trihydrate or amoxycillin triethylamine salt may be suspended or dissolved in the amide or a mixture of the amide and inert solvent such as methylene dichloride. This solution or suspension is then treated with at least one equivalent of a basic sodium salt, e.g. solid sodium 3-ethylhexanoate, or a solution of sodium methoxide or sodium hydroxide in an aliphatic alcohol such as methanol or isopropyl alcohol. Ambient temperatures may be used but lower temperatures (0-5°C) are preferred to minimize degradation.

The amides which form the amide solvate may be of formula (I):

R-CO-NR¹R² (I)

wherein R, R¹ and R² are the same or different and each represents hydrogen or a hydrocarbon group; or R and R¹ together represent a 3- or 4-carbon saturated or unsaturated chain.

The term 'hydrocarbon' includes groups having up to 18 carbon atoms, suitably up to 10 carbon atoms, conveniently up to 6 carbon atoms. Suitable hydrocarbon groups include C₁₋₆ alkyl, C₂₋₆ alkenyl, C₂₋₆ alkynyl, C₃₋₇ cycloalkyl, C₃₋₇ cycloalkyl (C₁₋₆)-alkyl, aryl, and aryl(C₁₋₆)alkyl.

Suitable alkyl groups include straight and branched chain alkyl groups containing from 1 to 6 carbon atoms, such as methyl, ethyl, propyl and butyl. A particular alkyl group is methyl.

A preferred aryl group is phenyl.

Preferably the amide is a tertiary amide, that is both R¹ and R² represent a hydrocarbon group.

Suitably R represents hydrogen or C₁₋₆ alkyl.

Preferably R represents hydrogen or methyl.

Suitably R¹ and R² are the same or different and each represents C₁₋₆ alkyl.

Preferably R¹ and R² represent methyl.

Alternatively R and R¹ together represent propylene (-(CH₂)₃-) or butylene (-(CH₂)₄-).

Suitable amides for inclusion in the amide solvate of sodium amoxycillin include dimethylformamide, dimethylacetamide, N-methylpyrrolidin-2-one or N-methylpiperidin-2-one.

In the process for the preparation of the crystalline sodium amoxycillin of this invention, the solvent molecules are removed from the sodium amoxycillin solvate. This removal should be carried out under conditions which do not degrade the penicillin. The method of removal of solvent molecules depends on the nature of the solvate. For many solvates, the solvent molecules are readily removable and the solvate free crystalline anhydrous sodium amoxycillin of the invention may be prepared by conventional drying means for example in vacuum. Conveniently the solvate is exposed to an adsorbent for the solvent molecules, either with or without vacuum which adsorbent may be a conventional drying agent such as phosphorus pentoxide, calcium chloride or silica.

Solvents which may be removed directly by such procedures include hydrocarbons, alkanols, ethers, chlorinated hydrocarbons and esters.

An alternative method for removal of solvent molecules, which is particularly useful for stable solvates, comprises the conversion of the solvate into a different solvate, from which the solvent molecules may be removed more readily, and subsequently removing the solvent molecules therefrom by conventional drying means. The conversion is conveniently carried out by dissolving or suspending the solvate in a non-aqueous solvent, such as an alkanol, or a mixture of non-aqueous solvents and adding a solvating solvent in excess.

This method is particularly useful for removing the amide from an amide solvate. Thus, the amide solvate of sodium amoxycillin is dissolved in a C₁₋₆ alkanol and a solvent of lower dielectric constant is added. The addition of the solvent of lower dielectric constant causes crystallisation of a solvate of sodium amoxycillin, from which the solvent molecules can be readily removed to produce crystalline anhydrous sodium amoxycillin of this invention.

Suitable C₁₋₆ alkanols include methanol and ethanol, preferably methanol. The initial dissolution of the amide solvate therein is suitably carried out at -40°C to +25°C, for example at -40°C to +10°C, preferably at -10°C to +10°C, conveniently at 0 to 5°C.

The sodium amoxycillin formed by the process of this invention is crystalline, usually in the form of prisms or rods. It is also anhydrous, typically containing less than 1% moisture and exhibits exceptional stability, undergoing no degradation at 80°C over a period of four hours. Unlike previously known spraydried or freeze-dried amorphous sodium amoxycillin, both of which are among the most hygroscopic of penicillins, this crystalline form is non-hygroscopic.

The infra-red spectrum (Nujol (Trademark) mull) of the crystalline sodium amoxycillin of this invention is shown in the accompanying Figure 1.

The crystalline sodium amoxycillin of this invention is also characterised by an X-ray powder diffractogram showing maxima within ± 0.1° of the values listed in Example 21 hereinafter.

A further advantage of the process for production of the sodium amoxycillin is that the process incorporates a step in which sodium amoxycillin is wholly in solution, so that the solution can be sterile filtered. This operation is important for a product which is to be employed for parenteral administration.

The crystalline form of the sodium amoxycillin can be reduced or removed without destroying its anhydrous nature, for example by mechanical degradation of the crystals or by dissolving in an anhydrous solvent. Similarly it is possible to retain the crystalline form of the sodium amoxycillin whilst allowing some hydration of the anhydrous state.

The present invention also provides a pharmaceutical composition which comprises crystalline sodium amoxycillin and a pharmaceutically acceptable carrier.

The compositions of the invention include those in a form adapted for oral, topical or parenteral use and may be used for the treatment of the infection in mammals including humans.

However the chief utility of sodium amoxycillin is for parenteral use.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilized before filling into a suitable vial or ampoule and sealing. Advantageously, agents such as a local anaesthetic, preservative and buffering agents can be dissolved in the vehicle. Alternatively the dry powder is sealed in a vial and an accompanying vial of water for injection may be supplied to reconstitute the liquid prior to use. Parental suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the composition comprises dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

Advantageously, the solution for injection prepared from the crystalline sodium amoxycillin also comprises at least one water-miscible pharmaceutically acceptable alcoholic compound such as ethanol, n-propanol, iso-propanol, ethoxyethanol, diethylene glycol, and propylene glycol.

A particularly advantageous alcohol for incorporation in the injectable composition is benzyl alcohol, which is incorporated at a level of up to 10%, preferably 1-5%, suitably about 3 % of the composition.

It is also advantageous to incorporate a basic excipient with the crystalline sodium amoxycillin in the compositions adapted for administration by injection. In particular, this invention also provides an injectable composition comprising crystalline sodium amoxycillin and trisodium phosphate. A suitable weight ratio of the crystalline sodium amoxycillin to the trisodium phosphate is from 8:1 to 50:1, preferably from 10:1 to 30:1, especially from 14:1 to 28:1. The inclusion of a base such as trisodium phosphate is particularly desirable to obtain rapid and complete dissolution of the sodium amoxycillin in aqueous solutions of high concentration required for intramuscular injection (typically 1 g of sodium amoxycillin 2 ml of water).

One advantageous presentation is a twin pack consisting of a vial containing crystalline sodium amoxycillin and a separate vial containing an aqueous solution of a buffer, for example trisodium phosphate.

The crystalline sodium amoxycillin may be the sole therapeutic agent in the compositions of the invention or a combination with other antibiotics or with a β-lactamase inhibitor may be employed.

Advantageously, the compositions also comprise a compound of formula (II) or a pharmaceutically acceptable salt or ester thereof: 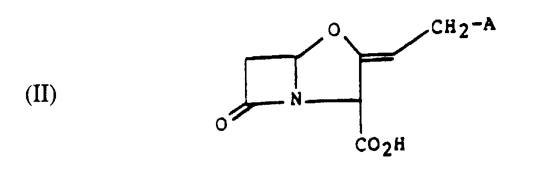 wherein A is hydroxyl, substituted hydroxyl, thiol, substituted thiol, amino, mono- or di-hydrocarbyl-substituted amino, or mono- or di-acylamino.

Preferably A represents hydroxy or methoxy, i.e. compound (II), preferably in the form of a salt, represents clavulanic acid or the methyl ether of clavulanic acid.

A preferred composition comprises crystalline sodium amoxycillin together with a salt of clavulanic acid, in particuiar the sodium or potassium salt, preferably potassium clavulanate. A particular composition of this invention comprises crystalline sodium amoxycillin, potassium clavulanate and trisodium phosphate.

A further advantageous composition comprises crystalline sodium amoxycillin together with a compound of formula (III) or a pharmaceutically acceptable salt or *in vivo* hydrolysable ester thereof: 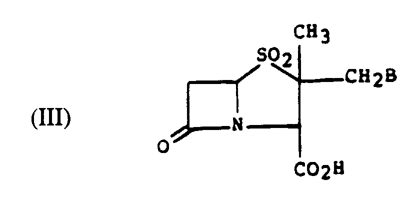 wherein B represents hydrogen or chloro.

The present invention also provides a method of treating bacterial infections in animals, in particular humans or domestic mammals, which comprises the administration of a composition of this invention.

The following Examples illustrate this invention.

### Preparation 1: Dimethylacetamide solvate of sodium amoxycillin

Dimethyl acetamide (200 ml) and triethylamine (26.1 ml, 0.186 mole) were cooled to 0°C and amoxycillin trihydrate (74.2 g; 0.177 mole) was added over 5 minutes at 0-5°C. The mixture was stirred for 5 minutes at 0 - 5°C, filtered, and sodium ethylhexanoate (33 g at 100%, 0.21 mole) dissolved in 60 ml methanol was added over 5 minutes at 0-5°C. The solution was stirred for 4 hours, warming to 20°C over the first hour. The product was filtered, washed with isopropyl alcohol (100 ml) and dried at 35°. The product weighed 75.2 g and assayed at 76% amoxycillin content (Theory 77%, 88% yield).

### Preparation 2: Dimethylacetamide solvate of sodium amoxycillin

The procedure of preparation 1 was repeated but using isopropyl alcohol (150 ml) in place of methanol to dissolve the sodium ethylhexanoate and gave 78.7 g product, amoxycillin content 72.9% (Yield 89%).

### Preparation 3: Dimethylformamide solvate of sodium amoxycillin

Dimethyl formamide (200 ml) and triethylamine (30 ml, 0.214 mole) were cooled to 5°C, and amoxycillin trihydrate, (43 g at 100%, 0.1012 mole) was added. The solution was cooled to 0-5°C and stirred 1/2 hour. Sodium 2-ethylhexanoate (24.7 g, 0.16 mole), dissolved in 150 ml isopropyl alcohol was added and the solution stirred 3 hours. The suspension was filtered and the cake washed with 100 ml isopropyl alcohol and 100 ml methylene dichloride. The product was dried at 35°. Yield of solvate 53 g; 94% assay by H.P.L.C.; 76.5% amoxycillin content (as free acid).

### Preparation 4: N-Methyl-2-pyrrolidinone solvate of sodium amoxycillin

N-methyl-2-pyrrolidinone (170 ml) and triethylamine (26.1 ml, 0.187 mole) were cooled to 0-5°C and amoxycillin trihydrate (74.2 g, 0.177 mole) was added over 10 minutes. The solution was stirred for one hour and filtered. Sodium ethylhexanoate (33.3 g, 0.2 mole) in 50 ml N-methyl-2-pyrrolidinone was then added. After stirring one hour, 400 ml methylene chloride was added over 30 minutes. The mixture was stirred for a further 3 hours at 20°C, filtered, and the product washed with 150 ml isopropyl alcohol. After drying at 35°, the product weighed 77g (86% yield) with an assay of 726 mcg. amoxycillin/mg.

### Example 1: Preparation of crystalline sodium amoxycillin from dimethtylacetamide solvate

### via tetrahydrofuran solvate

10g of the dimethylacetamide solvate of sodium amoxycillin as prepared in Preparation 1 was added to 60 ml methanol at 0-5°C and over one minute. The solution was stirred 25 minutes at 0 - 5°C and filtered. Tetrahydrofuran (150 ml) was added to the filtrate holding 0-5°C over 5 minutes, and the mixture stirred for 1½ hours at 0-5°. The suspension was filtered, the product washed with methylene chloride (75 ml), and dried in vacuo at 45° with a nitrogen bleed for 16 hours. The crystalline sodium amoxycillin (6.7 g, 83 % yield) assayed at 930 mcg/mg (amoxycillin free acid). The product contained 0.5% moisture.

### Example 2: Via toluene solvate

Preparation of the sodium amoxycillin-DMA solution as described in Example 1 and precipitating with toluene (200 ml, 1 hour) gave 5.5 g of crystalline product (66% yield). H.P.L.C. assay 920 mcg. amoxycillin/mg. The material contained 0.5% water.

### Example 3: Via isopropanol solvate

Preparation of the sodium amoxycillin-DMA solution as described in Example 1 above and precipitation with 400 ml isopropanol (1½ hours) gave 5.0 g crystalline product (56% yield; H.P.L.C. assay 837 mcg/mg).

### Example 4: Via isopropyl ether and toluene solvate

Preparation of the sodium amoxycillin-DMA solution as in Example 1 and precipitating with 150 ml toluene and then 90 ml isopropyl ether (at 20-25°) gave 8.0 g (96%) of crystalline sodium amoxycillin with an H.P.L.C. assay of 906 mcg. amoxycillin/mg.

### Example 5: Via n-propanol solvate

Preparation of the sodium amoxycillin-DMA solution as described in Example 1 above and precipitating with 200 ml n-propanol (1 hour) gave 4.6 g crystalline product (51% yield) at an assay of 825 mcg. amoxycillin/mg.

### Example 6: Via 1,2-dimethoxy ethane solvate

Preparation of the sodium amoxycillin-DMA solution as described in Example 1 above and precipitating with 100 ml 1,2-dimethoxy ethane (one hour) gave 7.5 g crystalline product (67% yield) with an H.P.L.C. assay of 666 mcg amoxycillin/mg.

### Example 7: Via methyl acetate solvate

Preparation of the sodium amoxycillin-DMA solution as described in Example 1 above and precipitating with 100 ml methyl acetate (1/ hours) gave 5.5 g crystalline sodium amoxycillin containing 836 mcg amoxycillin/mg.

### Example 8: Via dioxane solvate

Preparation of the sodium amoxycillin-DMA solution as described in Example 1 above and precipitating with 200 ml dioxane (1 hour) gave 6.6 g crystalline sodium amoxycillin (76% yield) containing 863 mcg amoxycillin/mg.

### Example 9: Via bis(2-methoxyethyl) ether solvate

Preparation of the sodium amoxycillin-DMA solution as described in Example 1 above and precipitating with 200 ml bis (2-methoxyethyl) ether (1½ hours) gave 7.4 g (79% yield) of crystalline sodium amoxycillin containing 800 mcg. amoxycillin/mg. (H.P.L.C.).

### Example 10: Via t-butanol solvate

Preparation of the sodium amoxycillin-DMA solution as described in Example 1 above and precipitating with 150 ml t-butanol (15 minutes) gave 6.9 g (74% yield) of crystalline sodium amoxycillin containing 802 mcg. amoxycillin/mg. (H.P.L.C.)

### Example 11: Via tetrahydrofuran solvate

Preparation of the sodium amoxycillin-DMA solution as described in Example 1 above but adding 6 ml water and precipitating with 200 ml tetrahydrofuran (15 minutes) gave 6.1 g (75% yield) of crystalline sodium amoxycillin containing 0.5% moisture and 927 mcg. amoxycillin/mg by H.P.L.C.

### Example 12: Via benzene solvate

Preparation of the sodium amoxycillin-DMA solution as described in Example 1 above and precipitating with 150 ml benzene (15 minutes) gave 2.0 g crystalline sodium amoxycillin with an assay of 918 mcg. amoxycillin/mg.

### Example 13: Via n-butanol solvate

Preparation of the sodium amoxycillin-DMA solution as described in Example 1 above and precipitating with 150 ml n-butanol gave 6.0 g crystalline product (66.2% yield) assaying at 827 mcg. amoxycillin/mg.

### Example 14: Via di-isopropyl ether solvate

Preparation of the Sodium Amoxycillin-DMA solution as above and precipitating with 150 ml di-isopropyl ether (10 minutes gave 7.5 g of crystalline sodium amoxycillin (84.3% Yield) at an assay of 843 mcg. amoxycillin/mg.

### Example 15: Via tetrahydrofuran solvate

Cool 60 ml methanol to 0-5°C and add sodium amoxycillindimethylacetamide solvate (10 g as is) over 30 seconds. Stir at 0-5°C for 25-30 minutes and filter. To the filtrate (0-5°C) add 150 ml tetrahydrofuran over 5 minutes and stir 1½ hours at 0-5°C. The suspension is filtered and the product washed with 100 ml tetrahydrofuran containing 2% water, followed by 50 ml methylene dichloride. The product is vacuum dried at 45 ° with a nitrogen bleed. The product, obtained by this procedure, crystalline sodium amoxycillin, weighed 7.2 g (89.3% yield) with 0.72% moisture and an H.P.L.C. assay of 931 mcg. amoxycillin/mg.

### Example 16: Via tetrahydrofuran solvate

Using the sodium amoxycillin- DMA solution described in Example 1 sodium amoxycillin was crystalised by adding tetrahydrofuran (150 ml) after further stirring for 10 minutes. After crystallisation had proceeded for 1½ hours the product was isolated, washed with tetrahydrofuran and methylene chloride and dried in vacuo at 45°C. Sodium amoxycillin (7.6 g; 93%) was thereby isolated as crystalline rods assaying at 919 mcg. Amoxycillin/mg by H.P.L.C. Moisture content was 0.67%.

### Example 17: Preparation of crystalline sodium amoxycillin from dimethylformamide solvate

### Via tetrahydrofuran solvate

To methanol (60 ml), cooled to 0-5°C was added 10.0 g sodium amoxycillin dimethylformamide solvate prepared as in Preparation 3. The solution was stirred 30 minutes at 0-5°C and filtered. To the filtrate at 0-5°C was added 150 ml tetrahydrofuran over 20 minutes. The suspension was stirred one hour at 0-5°C, filtered, and the product washed with 90 ml methylene chloride. After vacuum drying the crystalline product weighed 6.5 g (79.4% yield) assaying at 916 mcg. amoxycillin/mg by H.P.L.C.

### Example 18: Preparation of crystalline sodium amoxycillin from N-methyl-2-pyrrolidinone solvate Via toluene solvate

Methanol (60 ml) was cooled to 0-5°C and 10 g of the sodium amoxycillin-N-methyl-2-pyrrolidinone solvate prepared in Preparation 4 was added. The solution was stirred 25 minutes at 0-5°C and filtered, and the product washed with 50 ml methanol : toluene (1:5) and 50 ml methylene dichloride. The crystalline sodium amoxycillin after drying at 45° in vacuo with a nitrogen bleed weighed 4.0 g, (48% yield) and assayed at 909 mcg. amoxycillin/mg.

### Example 19: Via tetrahydrofuran solvate

Preparation of the sodium amoxycillin-N-methyl-2-pyrrolidinone solution as described in Example 18 but with precipitation by 150ml tetrahydrofuran and washing with tetrahydrofuran and methylene chloride gave 7.0g sodium amoxycillin (85% yield) containing 913 mcg. amoxycillin/mg.

### Example 20: Preparation of crystalline anhydrous sodium amoxycillin via tetrahydrofuran solvate

Amoxycillin trihydrate (4.19g, 0.010 mole) was added to a mixture of sodium-2-ethylhexanoate (1.70g, 0.0102 mole) and triethylamine (1.50 ml, 0.0108 mole) in methanol (30 ml) with stirring at room temperature. A clear solution was obtained after 15 minutes, and was treated with tetrahydrofuran (THF) (100ml) and seeded. Crystallisation began immediately. The mixture was cooled at ca 5°C for 0.75 hour, the product collected, washed with THF/methanol (5:1, 25 ml), then methylene chloride (50ml) and dried in vacuo over phosphorous pentoxide. Yield 2.74g (70%).

The infra-red spectrum (nujol mull) was indistinguishable from that of material prepared according to Example 1.

### Example 21: Preparation of crystalline anhydrous sodium amoxycillin via methyl acetate solvate

Amoxycillin trihydrate (83.8g) was suspended in methyl acetate (300ml) and a solution of sodium-2-ethyl hexanoate (36.0g) and triethylamine (35ml) in methanol (300 ml) added in one portion with vigorous stirring at room temperature. A virtually clear solution was obtained after 3 minutes. This was filtered, seeded, and diluted with methyl acetate (300ml) with vigorous stirring. As crystallization proceeded, more methyl acetate (total 600ml) was added in a slow stream over 30 minutes. The mixture was stirred for a further 15 minutes at room temperature, then stored at ca. 5°C for 2 hours.

The product was collected, slurried in methyl acetate/methanol (4:1, 250 ml), washed on the filter with methyl acetate/methanol (6:1, 100 ml) then with methyl acetate and finally with methylene chloride. The product was dried in low vacuum for 30 minutes. (The n.m.r. spectrum of material dried in this way showed the presence of approx. 1 mole of methyl acetate. Yield 65.5g

The solvated sodium amoxycillin was dried in high vacuum over phosphorus pentoxide until the expected weight loss had been achieved. Yield 55.3g Weight yield 71.4%

The material had the following assays:-

| | |
|---|---|
| Purity (Imidazole) | 96.4% as sodium salt |
| Iodine absorbing substances | 0.78% |
| Water (K.F.) | 0.17% |
| Solvents: | |
| Methylene chloride | 0.01 % |
| Methanol | 0.008% |
| Methyl acetate | 1.0% |

The product of Example 21 was examined by X-ray powder diffraction using a Phillips scanning diffractometer and Cu Kα radiation, set at 46 Kv and 35 mA, with a time constant of 0.3 seconds. Diffraction maxima were recorded from 30° to 8° 2θ. The following lines were observed.

| | |
|---|---|
| 29.8 | 18.9 |
| 29.2 | 17.8 |
| 28.4 | 17.1 |
| 28.0 | 16.5 |
| 26.5 | 15.8 |
| 25.9 | 15.5 |
| 25.7 | 15.0 |
| 25.2 | 14.0 |
| 24.0 | 13.5 |
| 23.0 | 12.5 |
| 22.3 | 11.3 |
| 21.4 | 10.5 |
| 20.9 | 9.4 |
| 20.1 | 8.5 |

### Example 22: Preparation of crystalline anhydrous sodium amoxycillin via methyl acetate solvate

8g of amoxycillin trihydrate (at 86%) was suspended in a mixture methanol/methyl acetate (50ml/50ml), precooled at -10°C. The product was dissolved by addition of a sodium methoxide solution (1.1g sodium methoxide dissolved in 10ml of methanol), maintaining the temperature between -8°C and-10°C. Precipitation was effected by a rapid addition (5 min.) of 150 ml methyl acetate increasing the temperature up to 0-5°C. A further 150 ml methyl acetate was added over 15 minutes and the suspension stirred for 2 hours, maintaining the temperature between 0-5°C. The product was isolated by filtration, washed with 2 x 40 ml methylene dichloride and dried at 60°C in a vacuum oven under nitrogen bleed, to give crystalline anhydrous sodium amoxycillin.

The average activity yield from 6 syntheses was 81.5%, with product purity between 89.1% and 92.3% (acid as is).

## Claims

1. Crystalline sodium amoxycillin, characterised in that the sodium amoxycillin is substantially free of solvent.

2. A process for the preparation of crystalline sodium amoxycillin according to claim 1, which process comprises removing the solvent molecules from a crystalline solvate of sodium amoxycillin, and wherein the said removal of solvent molecules from the solvate may comprise the conversion of the solvate into a different solvate, from which the solvent molecules may be removed more readily, and subsequently removing the solvent molecules therefrom.

3. A process according to claim 2 wherein the solvent molecules are removed by drying means.

4. A process according to claim 2, which process comprises removing the amide from an amide solvate of sodium amoxycillin.

5. A process according to claim 4 wherein the amide is of the formula (I):
R-CO-NR¹R² (I)
wherein R, R¹ and R² are the same or different and each represents hydrogen or a hydrocarbon group; or R and R¹ together represent a 3- or 4- carbon saturated or unsaturated hydrocarbon chain.

6. A process according to claim 5 wherein the amide is dimethylformamide, dimethylacetamide, N-methylpyrrolidin-2-one or N-methylpiperidin-2-one.

7. A process according to claim 4 wherein the amide is removed from the amide solvate of sodium amoxycillin by dissolving the amide solvate in a C₁₋₆ alkanol and adding a solvent of lower dielectric constant to cause crystallisation of a solvate of sodium amoxycillin in crystalline form. and removing the solvent molecules therefrom.

8. A pharmaceutical composition useful for treating bacterial infections in humans and animals which comprises an antibacterially effective amount of crystalline sodium amoxycillin according to claim 1 in combination with a pharmaceutically acceptable carrier.

9. A composition according to claim 8 which additionally contains trisodium phosphate.

10. A pharmaceutical composition useful for treating bacterial infections in humans and animals which comprises a β-lactamase inhibitor, and an antibacterially effective amount of a crystalline sodium amoxycillin according to claim 1, in combination with a pharmaceutically acceptable carrier.

11. A composition according to claim 10 wherein the β-lactamase inhibitor is a pharmaceutically acceptable salt of clavulanic acid.

12. The use of crystalline sodium amoxycillin according to claim 1, for the manufacture of a medicament for treatment of bacterial infections.

## Patentansprüche

1. Kristallines Natriumamoxycillin, dadurch gekennzeichnet,daß das Natriumamoxycillin im wesentlichen kein Lösungsmittel enthält.

2. Ein Verfahren zur Herstellung eines kristallinen Natriumamoxycillins nach Anspruch 1, welches Verfahren das Entfernen der Lösungsmittelmoleküle aus einem Solvat von Natriumamoxycillin umfaßt, und in welchem die besagte Entfernung der Lösungsmittelmoleküle aus dem Solvat die Umwandlung des Solvats in ein anderes Solvat umfassen kann, aus welchem die Lösungsmittelmoleküle schneller entfernt werden können, und welches anschließend das Entfernen der Lösungsmittelmokeküle aus diesem umfaßt.

3. Ein Verfahren nach Anspruch 2, in welchem die Lösungsmittelmoleküle durch Trocknungseinrichtungen entfernt werden.

4. Ein Verfahren nach Anspruch 2, welches Verfahren das Entfernen des Amids aus einem Amidsolvat von Natriumamoxycillin umfaßt.

5. Ein Verfahren nach Anspruch 4, in welchem das Amid die Formel(I) hat
R-CO-NR¹R² (I)
in welcher R, R¹ und R² gleich oder verschieden sein können und jeweils Wasserstoff oder eine Kohlenwasserstoffgruppe darstellen; oder R und R¹ zusammen eine gesättigte oder ungesättigte Kohlenwasserstoffkette mit 3 oder 4 Kohlenstoffatomen darstellen.

6. Ein Verfahren nach Anspruch 5, in welchem das Amid Dimethylformamid, Dimethylacetamid, N-Methylpyrrolidin-2-on oder N-Methylpiperidin-2-on ist.

7. Ein Verfahren nach Anspruch 4, in welchem das Amid aus dem Amidsolvat von Natriumamoxycillin durch Auflösen des Amidsolvats in einem C₁₋₆-Alkanol und Hinzufügen eines Lösungsmittels mit einer niedrigeren Dielektrizitätskonstante , um die Kristallisation eines Solvats von Natriumamoxycillin in kristalliner Form zu bewirken, und durch Entfernen der Lösungsmittelmoleküle ausdiesem entfernt wird.

8. Eine pharmazeutische Zusammensetzung zur Anwendung bei der Behandlung von bakteriellen Infektionen bei Mensch und Tier, welche eine antibakteriell wirksame Menge eines kristallinen Natriumamoxycillins nach Anspruch 1 in Verbindung mit einem pharmazeutisch verträglichen Träger enthält.

9. Eine Zusammensetzung nach Anspruch 8, welche zusätzlich Trinatriumphosphat enthält.

10. Eine pharmazeutische Zusammensetzung zur Verwendung bei der Behandlung von bakteriellen Infektionen bei Mensch und Tier, welche eine β-Laktamase hemmende Menge einer β-Laktamase-Hemmsubstanz und eine antibakteriell wirksame Menge eines kristallinen Natriumamoxycillins nach Anspruch 1 in Verbindung mit einem pharmazeutisch verträglichen Träger enthält.

11. Eine Zusammensetzung nach Anspruch 10, in welcher die β-Laktamase-Hemmsubstanz eine pharmazeutisch verträgliches Salz der Clavulansäure ist.

12. Verwendung von kristallinem Natriumamoxycillin nach Anspruch 1, für die Herstellung eines Medikaments zur Behandlung von bakteriellen Infektionen.

## Revendications

1. Amoxycilline de sodium cristalline, caractérisée en ce que l'amoxycilline sodique est pratiquement dépourvue de solvant.

2. Procédé pour la préparation d'amoxycilline de sodium cristalline suivant la revendication 1, caractérisé en ce qu'il comprend l'élimination des molécules de solvant à partir d'un solvat d'amoxycilline sodique.

3. Procédé suivant la revendication 2, caractérisé en ce que les molécules de solvant sont éliminées par séchage.

4. Procédé suivant la revendication 2, caractérisé en ce qu'il comprend l'élimination de l'amide à partir d'un solvat amidique d'amoxycilline sodique, laquelle élimination des molécules de solvant à partir de solvat peut comprendre la conversion dudit solvat en un solvat différent à partir duquel les molécules du solvant peuvent être éliminées plus facilement, suivie de l'élimination des molécules du solvant.

5. Procédé suivant la revendication 4, caractérisé en ce que l'amide est représenté par la formule (I) :
R-CO-NR¹R² (I)
dans laquelle R, R¹ et R² sont identiques ou différents et représentent chacun un atome d'hydrogène ou un groupe hydrocarboné, ou bien R et R¹ forment ensemble une chaîne hydrocarbonée saturée ou insaturée à 3 ou 4 atomes de carbone.

6. Procédé suivant la revendication 5, caractérisé en ce que l'amide est le diméthylformamide, le diméthylacétamide, la N-méthylpyrrolidin-2-one ou la N-méthylpipéridin2-one.

7. Procédé suivant la revendication 4, caractérisé en ce que l'amide est éliminé du solvat amidique d'amoxycilline sodique par dissolution du solvat amidique dans un alcanol en C₁₋₆ et addition d'un solvant de constante diélectrique inférieure pour provoquer la cristallisation d'un solvat d'amoxycilline sodique sous une forme cristalline et l'élimination des molécules de solvant de celui-ci.

8. Composition pharmaceutique utile pour le traitement d'infections bactériennes chez les hommes et les animaux, caractérisée en ce qu'elle comprend une quantité efficace du point de vue antibactérien d'amoxycilline de sodium cristalline suivant la revendication 1, en association avec un support acceptable du point de vue pharmaceutique.

9. Composition suivant la revendication 8, caractérisé en ce qu'elle contient de plus du phosphate trisodique.

10. Composition pharmaceutique utile pour le traitement d'infections bactériennes chez les hommes et les animaux, caractérisée en ce qu'elle comprend une quantité inhibant la bêta-lactamase d'un inhibiteur de bêta-lactamase et une quantité efficace du point de vue antibactérien d'une amoxycilline de sodium cristalline suivant la revendication 1, en association avec un support acceptable du point de vue pharmaceutique.

11. Composition suivant la revendication 10, caractérisée en ce que l'inhibiteur de bêta-lactamase est un sel d'acide clavulanique acceptable du point de vue pharmaceutique.

12. Utilisation d'amoxycilline de sodium cristalline suivant la revendication 1, caractérisée en ce qu'elle concerne la préparation d'un médicament pour le traitement d'infections bactériennes.
